# FASCICULE DE BREVET EUROPEEN

(11) **EP 2 100 202 B1**
(45) Date de publication et mention de la délivrance du brevet: **13.02.2013**
(21) Numéro de dépôt: 07870383.2
(22) Date de dépôt: 03.12.2007
(51) Int. Cl.: G05D 21/02, C12M 1/40, G05D 21/00, F03G 7/00

(54) **Applications d'un dispositif de variation du PH d'une solution**
Anwendungen einer Vorrichtung zur Veränderung des pH-Wertes einer Lösung
Applications of a device for changing the ph of a solution

(30) Priorité: 04.12.2006 FR 0655296
(43) Date de publication de la demande: 16.09.2009
(60) Demande divisionnaire: 11183185.5
(73) Titulaire: Université Joseph Fourier, 38041 Grenoble Cedex 9 (FR); Institut National des Sciences Appliquées de Toulouse, 31077 Toulouse Cedex 4 (FR)
(72) Inventeur: CINQUIN, Philippe, 38330 Saint Nazaire Les Eymes (FR); LENOUVEL, François, 38100 Grenoble (FR); DURRIEU, Vanessa, 31400 Toulouse (FR); MATHE, Stéphane, 31500 Toulouse (FR); MOZER, Pierre, 94300 Vincennes (FR)
(74) Mandataire: de Beaumont, Michel
(86) Numéro de dépôt international: PCT/FR2007/052435
(87) Numéro de publication internationale: WO 2008/074958

(56) Documents cités:
- DE-C1- 19 728 663
- DE-C2- 4 218 937
- FR-A1- 2 881 481

## Description

### Domaine de l'invention

La présente invention concerne des applications d'un dispositif de variation du pH d'une solution.

### Exposé de l'art antérieur

Pour certaines applications, il est souhaitable de pouvoir modifier le pH d'une solution. Un exemple d'application concerne la régulation d'un phénomène physico-chimique se produisant en solution et dont la vitesse varie en fonction du pH de la solution, par exemple, la variation de solubilisation d'une substance dans une solution dont on fait varier le pH. Un autre exemple d'application concerne la régulation de propriétés chimiques ou mécaniques d'une solution qui varient en fonction du pH. Il s'agit, par exemple, de l'osmolarité de la solution.

La modification du pH d'une solution initiale peut être obtenue, de façon classique, en mélangeant à la solution initiale une solution supplémentaire acide ou basique. Le pH de la solution finalement obtenue après mélange se stabilise à une valeur qui dépend du pH de la solution initiale, du pH de la solution supplémentaire et des volumes des solutions initiale et supplémentaire.

Toutefois, pour certaines applications, il serait souhaitable de pouvoir modifier le pH d'une solution sans devoir y ajouter une solution acide ou basique. A titre d'exemple, il serait souhaitable de pouvoir modifier le pH d'une solution contenue dans le corps humain, appelée par la suite solution biologique, en limitant le plus possible toute intervention au niveau du corps humain pour réaliser la modification de pH.

### Résumé de l'invention

La présente invention vise des applications d'un dispositif de variation du pH d'une solution qui ne nécessitent pas l'ajout d'une solution acide ou basique et plus particulièrement d'un dispositif de variation de pH susceptible d'être implanté au niveau du corps humain.

Pour atteindre ces objets, la présente invention prévoit des applications d'un dispositif de variation du pH d'un solvant dans lequel est dissous une substance tel que définies dans les revendications ci-après, le dispositif de variation du pH étant par exemple du type décrit dans DE-197 28 663.

### Brève description des dessins

Ces objets, caractéristiques et avantages, ainsi que d'autres de la présente invention seront exposés en détail dans la description suivante d'exemples de réalisation particuliers faite à titre non-limitatif en relation avec les figures jointes parmi lesquelles :
la figure 1 représente un premier exemple de réalisation d'un dispositif de variation de pH ;
la figure 2 représente un second exemple de réalisation du dispositif de variation de pH ;
les figures 3A et 3B représentent deux étapes de fonctionnement d'un exemple de réalisation d'un actionneur osmotique selon l'invention mettant en oeuvre le dispositif de variation de pH selon le premier exemple de réalisation ;
les figures 4A à 4D représentent quatre étapes de fonctionnement d'un premier exemple de réalisation d'un moteur osmotique selon l'invention, mettant en oeuvre le premier exemple de réalisation du dispositif de variation de pH;
les figures 5A et 5B représentent deux étapes de fonctionnement d'un second exemple de réalisation d'un moteur osmotique selon l'invention, mettant en oeuvre le second exemple de réalisation du dispositif de variation de pH;
les figures 6A et 6B représentent deux étapes de fonctionnement d'une variante du second exemple de réalisation du moteur osmotique ;
les figures 7A et 7B représentent deux étapes de fonctionnement d'un dispositif d'épuration selon l'invention mettant en oeuvre le premier exemple de réalisation du dispositif de variation de pH;
les figures 8A à 8D représentent quatre étapes de fonctionnement d'un troisième exemple de réalisation d'un moteur osmotique selon l'invention, mettant en oeuvre le premier exemple de réalisation du dispositif de variation de pH ;
les figures 9A et 9B représentent deux étapes de fonctionnement d'un premier exemple de réalisation d'une pile électrique selon l'invention mettant en oeuvre le premier exemple de réalisation du dispositif de variation de pH ;
la figure 10 représente un seconde exemple de réalisation d'une pile électrique selon l'invention mettant en oeuvre le premier exemple de réalisation du dispositif de variation de pH ; et
la figure 11 illustre un procédé de traitement selon l'invention.

### Description détaillée

De mêmes éléments ont été désignés par les mêmes références aux différentes figures. Pour des raisons de clarté, seuls les éléments qui sont utiles à la compréhension de l'invention ont été représentés aux figures et seront décrits par la suite.

Il est prévu de favoriser une première série ou une seconde série de réactions chimiques dans une solution dont on souhaite modifier le pH, la première série de réactions chimiques entraînant une diminution du pH de la solution tandis que la seconde série de réactions chimiques entraîne une augmentation du pH de la solution.

Toute réaction aboutissant à la formation d'ions H⁺, et donc à une diminution du pH, peut convenir pour la première série de réactions. C'est en particulier le cas de l'oxydation du D-glucose, ou stéréoisomère D du glucose, par l'enzyme glucose oxydase, qui aboutit à la formation d'acide gluconique (susceptible de libérer un ion H⁺) et d'eau oxygénée. L'eau oxygénée est de plus dégradable par l'enzyme catalase ou l'enzyme peroxydase pour fournir des ions H⁺ supplémentaires. Les réactions mises en oeuvre sont les suivantes :

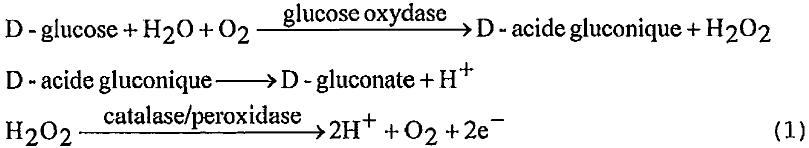

La première série de réactions peut correspondre à l'oxydation du L-glucose, ou stéréoisomère L du glucose, par l'enzyme L-Fucose déshydrogénase. Les réactions mises en oeuvre sont les suivantes : 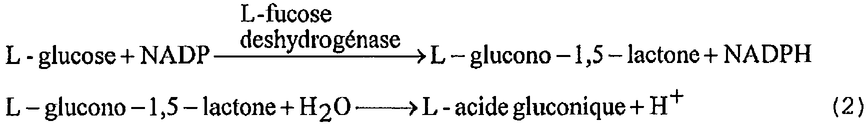 où le composé NADP correspond au Nicotinamide Adénine Dinucléotide Phosphate et où le composé NADPH correspond au même composé une fois réduit.

La seconde série de réactions mises en oeuvre pour rendre basique une solution peut correspondre à la dégradation de l'urée par l'enzyme uréase qui aboutit à la formation d'ions ammonium NH₄⁺ et d'ions hydroxyle OH⁻, c'est-à-dire à une augmentation du pH. Les réactions mises en oeuvre sont les suivantes :

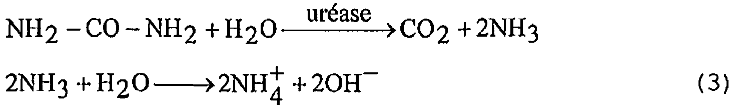

Les réactions (1) et (3) présentent l'avantage de pouvoir être directement mises en oeuvre avec une solution biologique qui contient naturellement du D-glucose, qui est le glucose participant à la glycémie, et de l'urée. Les réactions (2) peuvent facilement être mises en oeuvre avec une solution biologique qui contient naturellement le composé NADP. Il suffit alors d'ajouter du L-glucose à la solution biologique.

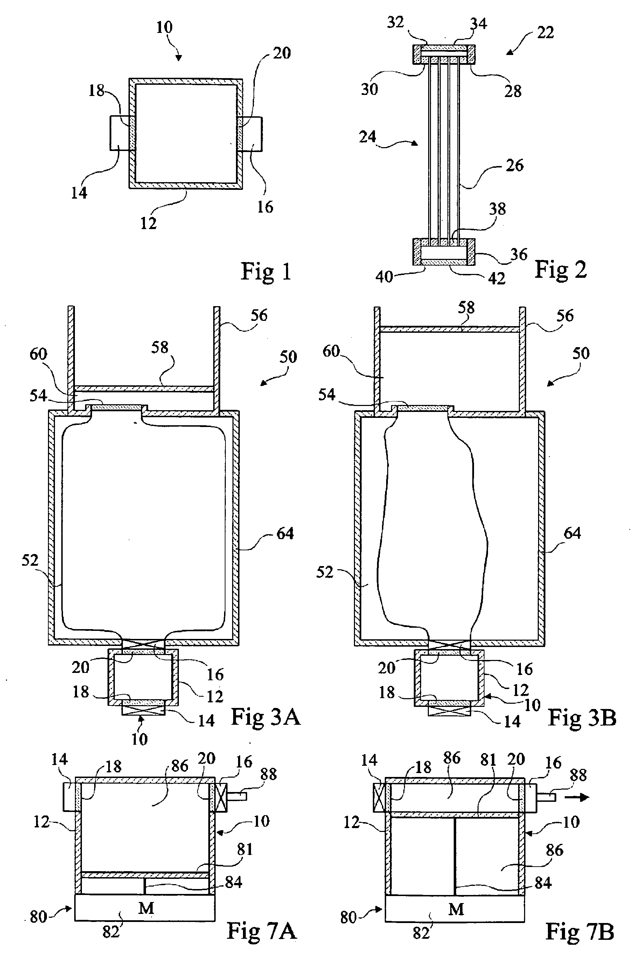

La figure 1 représente un premier exemple de réalisation d'un dispositif 10 de variation de pH. Le dispositif 10 est disposé dans une solution dont on souhaite modifier le pH, par exemple une solution biologique. Le dispositif 10 comprend une enceinte 12 étanche et une vanne 14 qui, lorsqu'elle est ouverte, met en communication le contenu de l'enceinte 12 avec la solution extérieure. Le dispositif 10 peut être relié à un système, non représenté, dont le fonctionnement nécessite l'apport d'une solution ayant un pH compris dans une plage déterminée. Pour ce faire, le dispositif 10 comprend une vanne 16 qui, lorsqu'elle est ouverte, met en communication le contenu de l'enceinte 12 avec le système à alimenter. Le dispositif 10 comprend une membrane 18 disposée entre la vanne 14 et l'enceinte 12 et une membrane 20 disposée entre la vanne 16 et l'enceinte 12. Les membranes 18 et 20 ont des seuils de coupure donnés, exprimés généralement en daltons, c'est-à-dire qu'elles laissent passer des particules ayant une masse moléculaire sensiblement inférieure au seuil de coupure.

Lorsqu'on souhaite acidifier la solution contenue dans l'enceinte 12, on peut favoriser dans l'enceinte 12 la réaction d'oxydation du glucose, par exemple selon les réactions (1). Pour ce faire, le dispositif 10 est placé dans une solution contenant du D-glucose, par exemple une solution biologique, et on dispose dans l'enceinte 12 des enzymes glucose oxydase et des enzymes catalase ou peroxydase. Les membranes 18 et 20 ont des seuils de coupure tels qu'elles permettent de retenir les enzymes glucose oxydase et les enzymes catalase ou peroxydase dans l'enceinte 12. En outre, la membrane 18 a un seuil de coupure suffisamment élevé pour laisser passer le glucose. A titre d'exemple, les membranes 18 et 20 ont un seuil de coupure de l'ordre de quelques centaines de Daltons.

Le fonctionnement du premier exemple de dispositif 10 va maintenant être décrit pour une opération de diminution du pH. Initialement, la vanne 16 est fermée et la vanne 14 est ouverte permettant la diffusion du D-glucose dans l'enceinte 12. La vanne 14 est alors fermée. Le D-glucose contenu dans l'enceinte 12 est alors oxydé pour fournir des ions H⁺ et de l'acide gluconique selon les réactions (1) précédemment décrites. Le pH de la solution contenue dans l'enceinte 12 diminue donc. La solution acide obtenue peut alors être utilisée par le système relié à l'enceinte 12 en ouvrant la vanne 16. La demanderesse a mis en évidence que pour une solution aqueuse ayant une concentration en D-glucose initiale de 5,5 mmol/l, ce qui correspond à la concentration moyenne de glucose dans le corps humain, une concentration d'enzyme glucose oxydase de 1 mg.ml⁻¹ soit 47 unités et un pH initial de 7, on obtient dans l'enceinte 12 un pH égal à 5 en l'espace d'une heure et un pH de 3,5 après trois heures.

L'acide gluconique issu de l'oxydation du glucose tend à former des sels, ou gluconate, avec les substances présentes en solution. S'il n'est pas souhaitable que le gluconate pénètre au niveau du système relié au dispositif 10, on choisit la membrane 20 avec un seuil de coupure suffisamment bas pour retenir le gluconate dans l'enceinte 12 lorsque la vanne 16 est ouverte. Le seuil de coupure de la membrane 20 est alors de l'ordre de 100 Daltons. A la prochaine ouverture de la vanne 14, le gluconate diffuse hors de l'enceinte 12. Dans le cas d'une application médicale pour laquelle le dispositif 10 est placé dans le corps humain, il est souhaitable que les enzymes catalase ou peroxydase soient disposées au niveau de la membrane 18 pour éviter le rejet dans le corps humain de radicaux libres issus de l'oxydation du D-glucose. Par ailleurs, le rejet de gluconate dans le corps humain ne présente pas de danger puisqu'il est naturellement évacué par les reins.

Pour une opération d'acidification d'une solution, on peut disposer dans l'enceinte 12 des enzymes L-fucose déshydrogénase, retenues par les membranes 18 et 20. Le dispositif 10 est alors placé dans une solution contenant du L-glucose, le fonctionnement du dispositif étant identique à ce qui a été décrit précédemment. Une solution biologique humaine ne comporte pas naturellement de L-glucose. Dans le cas où le dispositif 10 est placé dans le corps humain, on peut prévoir une étape d'ajout de L-glucose à la solution biologique, par exemple par injection par voie intraveineuse.

Le dispositif 10 selon le premier exemple de réalisation peut également être mis en oeuvre pour obtenir une augmentation de pH. Le dispositif 10 est alors disposé dans une solution contenant de l'urée, par exemple une solution biologique, et on favorise, dans l'enceinte 12, la dégradation de l'urée en ammoniaque et en acide carbonique selon les réactions (3). L'ammoniaque réagissant avec l'eau pour fournir des ions ammonium, on obtient ainsi une augmentation du pH. Pour ce faire, on dispose des enzymes uréase dans l'enceinte 12. Dans ce cas, les membranes 18 et 16 ont un seuil de coupure suffisamment bas pour retenir les enzymes uréase dans l'enceinte 12. En outre, la membrane 18 a un seuil de coupure suffisamment élevé pour laisser passer l'urée qui a une masse molaire de 60 g/mol. A titre d'exemple, la membrane 18 a un seuil de coupure de quelques centaines de Daltons. Le cycle de fonctionnement du dispositif 10 est alors identique à ce qui a été précédemment décrit. La demanderesse a mis en évidence que pour une solution ayant une concentration en urée de 3 mmol/l, ce qui correspond à la concentration moyenne d'urée dans le corps humain, et un pH initial de 7, on obtient dans l'enceinte 12 un pH égal à 8,2 en l'espace de deux heures et un pH de 9,2 après une nuit. Dans le cas d'une application médicale pour laquelle le dispositif 10 est placé dans le corps humain, le dioxyde de carbone issu des réactions (3) est naturellement évacué par la respiration. En outre, les ions ammonium issus des réactions (3) sont métabolisés par le foie.

La figure 2 représente un second exemple de réalisation d'un dispositif 22 de variation de pH. Le dispositif 22 comprend une enceinte 24 constituée d'un faisceau de fibres creuses à paroi semi-perméable 26, par exemple du type utilisé pour les opérations de dialyse. Chaque fibre a, par exemple, un diamètre de l'ordre de 200 µm. Le faisceau de fibres 26 est maintenu à une première extrémité par une première bague de jonction 28, par exemple par l'intermédiaire d'une zone de collage 30. La bague 28 comprend une ouverture 32 fermée par un bouchon 34. La seconde extrémité du faisceau de fibre 26 est maintenue par une seconde bague de jonction 36, par exemple par l'intermédiaire d'une zone de collage 38. La seconde bague 36 comprend une ouverture 40 fermée par un bouchon 42.

Le dispositif 22 est destiné à être plongé dans la solution dont on souhaite modifier le pH. Le diamètre et la longueur des fibres sont adaptés selon la surface d'échange désirée entre le faisceau de fibres 26 et la solution dans laquelle le dispositif 22 est plongé. Si l'on souhaite diminuer le pH, on dispose dans les fibres 26 des enzymes glucose oxydase et des enzymes catalase ou peroxydase par l'intermédiaire des ouvertures 32, 40 qui sont ensuite refermées par les bouchons 34, 42. La paroi des fibres 26 a alors un seuil de coupure tel qu'elle ne laisse pas passer les enzymes glucose oxydase et les enzymes catalase ou peroxydase qui sont retenues dans le faisceau de fibres 26 tandis qu'elle permet le passage du D-glucose et du gluconate. Si l'on souhaite augmenter le pH, on dispose dans les fibres 26 des enzymes uréase par l'intermédiaire des ouvertures 32, 40 qui sont ensuite refermées par les bouchons 34, 42. La paroi des fibres 26 a alors un seuil de coupure tel qu'elle ne laisse pas passer les enzymes uréase qui sont retenues dans le faisceau de fibres 26 tandis qu'elle permet le passage de l'urée.

Lorsque le dispositif 22 est implanté dans le corps humain pour acidifier une solution biologique, les enzymes catalase ou peroxydase sont de préférence fixées au niveau de la paroi interne des fibres 26 tandis que l'enzyme glucose oxydase peut être laissée libre dans le faisceau de fibres 26. Un tel agencement permet d'éviter que les radicaux libres créés par la dégradation du glucose par les enzymes D-glucose oxydase traversent la paroi des fibres 26 et se répandent dans la solution biologique.

Lorsque le dispositif 10, ou le dispositif 22, est utilisé pour acidifier une solution en mettant en oeuvre l'oxydation du D-glucose, il permet parallèlement la suppression d'une partie du D-glucose contenu dans la solution. En effet, les réactions mises en oeuvre dans l'enceinte 12 ou dans le faisceau de fibres 26 (réactions (1)) conduisent à la transformation du glucose en acide gluconique qui donne du gluconate. Un tel dispositif 10 (ou 22) peut être implanté dans le corps humain afin de supprimer un excès de D-glucose, généralement associé à un diabète de type I et de type II ou à une surcharge pondérale. Plusieurs lieux d'implantation du dispositif 10 (ou 22) sont alors envisageables. A titre d'exemple, le dispositif 10 (ou 22) peut être placé au niveau du péritoine ou d'un espace péri-cellulaire graisseux. Une implantation à proximité du péritoine présente l'avantage d'une riche vascularisation et d'une concentration élevée en D-glucose. Une implantation dans un espace graisseux diminue les risques de réactions inflammatoires. Le dispositif 10 (ou 22) peut également être implanté au niveau d'un muscle, par une ponction réalisée au moyen d'un trocart médical.

Dès son implantation dans le corps humain, le dispositif 22 transforme quotidiennement une certaine quantité de D-glucose en gluconate qui est naturellement éliminé par le rein. Le dispositif 10 permet, quant à lui, par la commande des vannes 14, 16, de contrôler la quantité de D-glucose transformée en gluconate. Par l'intermédiaire des vannes 14, 16 (respectivement des ouvertures 32, 40), il est possible d'accéder au contenu de l'enceinte 12 (respectivement des fibres 26) de façon à renouveler la population enzymatique contenue dans l'enceinte 12 (respectivement dans les fibres 26) si cela est nécessaire. Les chambres implantables utilisées classiquement pour les chimiothérapies permettent une ponction percutanée très facile, grâce à laquelle ce renouvellement peut être réalisé. Ceci permet de pallier une baisse d'activité enzymatique. Par ailleurs, l'accès au contenu de l'enceinte 12 (respectivement des fibres 26) permet également de réguler la population enzymatique de façon à contrôler la quantité de glucose consommée.

Les figures 3A et 3B représentent un exemple de réalisation d'un actionneur osmotique 50 selon l'invention mettant en oeuvre le premier exemple de réalisation du dispositif 10 de variation de pH. L'actionneur osmotique 50 comprend une enveloppe 52 déformable et étanche remplie d'un solvant et reliée au dispositif 10 au niveau de la vanne 16. L'enveloppe 52 est par ailleurs reliée par l'intermédiaire d'une membrane 54 à un corps cylindrique 56 dans lequel peut coulisser un piston mobile 58. Le piston mobile 58 et le corps cylindrique 56 définissent une chambre de dilatation 60. L'enveloppe 52 peut être disposée dans un boîtier rigide 64 qui est perforé pour que les déformations de l'enveloppe 52 ne soient pas gênées par un vide qui pourrait s'installer entre l'enveloppe 52 et le boîtier 64, si ce dernier était étanche.

La chambre de dilatation 60 contient une substance A, dissoute dans le solvant et osmotiquement active et à une concentration permettant un équilibre osmotique avec l'intérieur de l'enveloppe 52. La membrane 54 a un seuil de coupure suffisamment faible pour bloquer la substance A. A titre d'exemple, la substance A est le dextrane. Le dispositif 10 de variation de pH est du type permettant de rendre basique la solution qu'il contient, comme cela a été décrit précédemment. L'enveloppe 52 contient une substance Z osmotiquement active. La membrane 54 a un seuil de coupure suffisamment faible pour bloquer la substance Z. A titre d'exemple, la substance Z est le chitosane, soluble dans l'eau à pH acide, et insoluble à pH basique. La variation du pH de la solution entraîne donc la variation du nombre de molécules en solution et par conséquent de l'osmolarité de la solution.

Le fonctionnement de l'actionneur osmotique 50 selon l'invention va maintenant être décrit. Il s'agit d'un actionneur à un seul coup.

La figure 3A représente l'actionneur osmotique 50 dans l'état qui précède le fonctionnement de l'actionneur. La vanne 16 est alors fermée. L'enveloppe 52 a un volume maximum tandis que la chambre de dilatation 60 a un volume minimum. Les concentrations des substances A et Z sont initialement choisies de sorte que les pressions osmotiques dans la chambre de dilatation 60 et dans l'enveloppe 52 s'équilibrent, le piston 58 restant fixe. Avant le fonctionnement de l'actionneur 50, le dispositif 10 de variation de pH est commandé, comme cela a été décrit précédemment, de sorte que l'enceinte 12 contient une solution basique.

Lorsqu'on souhaite faire fonctionner l'actionneur osmotique 50, la vanne 16 est ouverte. Les ions OH⁻ contenus dans l'enceinte 12 se répandent dans l'enveloppe 52 entraînant une augmentation du pH du solvant contenu dans l'enveloppe 52. Lorsque le pH dans l'enveloppe 52 a augmenté jusqu'à la valeur souhaitée, la vanne 16 est refermée. La précipitation de la substance Z est alors favorisée. La pression osmotique dans l'enveloppe 52 diminue par rapport à la pression osmotique dans la chambre de dilatation 60 qui ne varie pas. Un transfert de solvant se produit donc, du solvant passant de l'enveloppe 52 à la chambre de dilatation 60 en traversant la membrane 54, entraînant le déplacement du piston 58. Le piston 58 est en phase ascendante.

La figure 3B représente l'actionneur osmotique 50 à la fin de la phase ascendante du piston 58. La chambre de dilatation 60 a alors un volume maximum. Le piston 58 peut être relié à un élément extérieur auquel on souhaite transmettre une énergie mécanique.

Dans l'exemple d'actionneur 50 décrit précédemment, le dispositif 10 est du type permettant de rendre basique la solution qu'il contient. Toutefois, l'actionneur 50 peut également être mis en oeuvre avec un dispositif 10 adapté à rendre acide la solution qu'il contient. Dans ce cas, initialement, la chambre de dilatation 60 peut avoir un volume maximum et l'enveloppe 52 un volume minimum comme cela est représenté en figure 3B. Lorsque la vanne 16 est ouverte, le pH du solvant dans l'enveloppe 52 et la chambre de dilatation 60 diminue, favorisant la solubilisation de la substance Z présente dans l'enveloppe 52. La pression osmotique dans l'enveloppe 52 augmente par rapport à la pression osmotique dans la chambre de dilatation 60 qui ne varie pas. Un transfert de solvant se produit donc, du solvant passant de la chambre de dilatation 60 dans l'enveloppe 52 en traversant la membrane 54, entraînant, par succion, le déplacement du piston 58. Le piston 58 est alors en phase descendante jusqu'à ce que la chambre de dilatation 60 ait un volume minimum comme cela est représenté en figure 3A.

Un exemple d'application de l'actionneur 50 consiste au gonflage d'un joint associé à une endoprothèse correspondant à un petit ressort, ou stent, glissé dans une cavité du corps humain (artère, par exemple) pour la maintenir ouverte. En effet, des joints peuvent être prévus aux extrémités du stent et être éventuellement gonflés, après la pose du stent, au moyen de l'actionneur 50 pour maintenir l'étanchéité aux extrémités du stent dans le cas d'une déformation ultérieure et indésirable de la cavité contenant le stent.

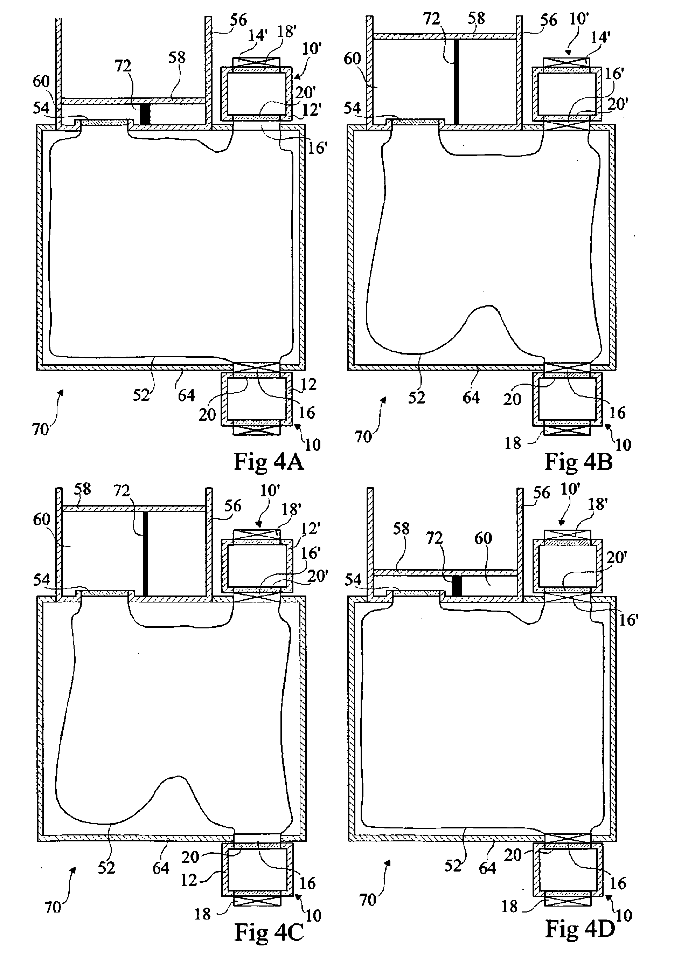

Les figures 4A à 4D représentent un premier exemple de réalisation d'un moteur osmotique 70 selon l'invention. Le moteur osmotique 70 inclut certains composants de l'actionneur 50 représenté aux figures 3A et 3B et les références correspondantes sont conservées. Un moyen de rappel 72, par exemple un ressort, exerce sur le piston 58 un effort de traction tendant à le ramener dans une position de repos. Le moteur 70 comprend deux dispositifs de variation de pH, notés 10 et 10'. Dans la suite de la description, on utilise des références primes pour désigner les éléments du dispositif de variation du pH 10', de façon à les distinguer des éléments du dispositif de variation de pH 10. L'enveloppe 52 est reliée au dispositif 10' au niveau de la vanne 16'. Le dispositif 10 est du type permettant la diminution du pH de la solution qu'il contient et le dispositif 10' est du type permettant l'augmentation du pH de la solution qu'il contient.

L'enveloppe 52 contient, tel que décrit précédemment, la substance Z susceptible de se solubiliser en présence d'ions H⁺, la réaction inverse selon laquelle la substance Z précipite étant susceptible de se produire en présence d'ions OH⁻. Comme cela a été décrit précédemment pour l'actionneur osmotique 50, la chambre de dilatation 60 contient une substance A, dissoute dans le solvant, osmotiquement active. La membrane 54 a un seuil de coupure suffisamment faible pour empêcher le passage des substances A et Z.

Un cycle de fonctionnement du moteur 70 selon l'invention va maintenant être décrit.

La figure 4A représente le moteur 70 au début d'un cycle. L'enveloppe 52 a un volume maximum et la chambre 60 a un volume minimum. Les concentrations des substances A et Z sont initialement choisies de sorte que les pressions osmotiques dans la chambre de dilatation 60 et dans l'enveloppe 52 s'équilibrent, le piston 58 restant fixe. Parallèlement au cycle d'ouvertures des vannes 16 et 16' qui va maintenant être décrit, les dispositifs 10, 10' sont commandés pour que les enceintes 12, 12' contiennent des solutions ayant le pH désiré.

La vanne 16 est fermée et la vanne 16' est ouverte. Les ions OH⁻ se répandent dans l'enveloppe 52 entraînant une augmentation du pH du solvant contenu dans l'enveloppe 52 et dans la chambre de dilatation 60. Lorsque le pH dans l'enveloppe 52 a augmenté jusqu'à la valeur souhaitée, la vanne 16' est fermée. La précipitation de la substance Z est alors favorisée. La pression osmotique dans l'enveloppe 52 diminue par rapport à la pression osmotique dans la chambre de dilatation 60 qui ne varie pas. Un transfert de solvant se produit donc, du solvant passant de l'enveloppe 52 à la chambre de dilatation 60 en traversant la membrane 54, entraînant le déplacement du piston 58. Le piston 58 est en phase ascendante.

La figure 4B représente le moteur 70 à la fin de la phase ascendante du piston 58. La chambre 60 a alors un volume maximum.

En figure 4C, on a ouvert la vanne 16 de façon à mettre en communication le contenu de l'enveloppe 52 avec le contenu de l'enceinte 12. Des ions H⁺ se répandent dans l'enveloppe 52 entraînant une diminution du pH du solvant contenu dans l'enveloppe 52 ainsi que dans la chambre de dilatation 60. Lorsque le pH est suffisamment acide, la vanne 16 est fermée. La solubilisation de la substance Z est alors favorisée, entraînant l'augmentation du nombre de particules osmotiquement actives en solution dans l'enveloppe 52. La pression osmotique augmentant dans l'enveloppe 52, un nouveau transfert de solvant se produit, du solvant passant de la chambre de dilatation 60 vers l'enveloppe 52 par l'intermédiaire de la membrane 54. L'action du ressort 72 favorise l'évacuation du solvant de la chambre de dilatation 60. Toutefois, le ressort 72 pourrait ne pas être présent, le piston 58 se déplaçant seulement par succion. Le piston 58 est dit en phase descendante.

La figure 4D représente le moteur 70 à la fin de la phase descendante du piston 58, ce qui clôt le cycle. Les concentrations des substances Z et A respectivement dans l'enveloppe 52 et la chambre de dilatation 60 sont alors sensiblement identiques aux concentrations en début de cycle.

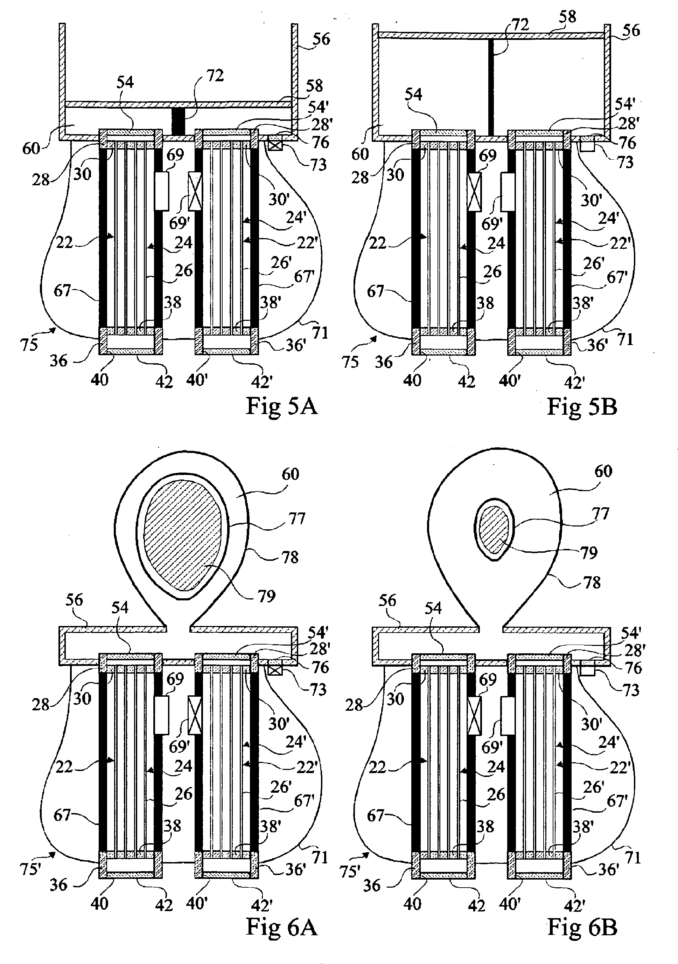

Les figures 5A et 5B représentent un second exemple de réalisation d'un moteur osmotique 75 mettant en oeuvre le second exemple de réalisation du dispositif 22 de variation de pH et certains éléments du moteur osmotique 70. Le moteur 75 comprend deux dispositifs de variation de pH, notés 22 et 22'. Dans la suite de la description, on utilise des références primes pour désigner les éléments du dispositif de variation du pH 22', de façon à les distinguer des éléments du dispositif de variation de pH 22. Chaque dispositif de variation de pH 22, 22' communique avec la chambre de dilatation 60 au niveau de la membrane 54, 54' qui remplace le bouchon 34. En outre, le dispositif de variation de pH 22 est contenu dans une enceinte 67 étanche qui relie les bagues de jonction 28 et 36 et le dispositif de variation de pH 22' est contenu dans une enceinte 67' étanche qui relie les bagues de jonction 28' et 36'. Une vanne 69, 69' est prévue au niveau de l'enceinte 67, 67'. Les enceintes 67, 67' sont contenues dans une enveloppe 71 semi-perméable qui est reliée au corps cylindrique 56 et aux bagues de jonction 36, 36' des dispositifs de variation de pH 22, 22'. Le corps cylindrique 56 comprend une vanne d'évacuation 73 qui relie la chambre de dilatation avec l'extérieur du moteur 75 par l'intermédiaire d'une membrane 76.

A titre d'exemple, le dispositif 22' est du type permettant la diminution du pH de la solution dans laquelle il est disposé selon les réactions (1) décrites précédemment, des enzymes glucose oxydase étant disposées dans les fibres 26'. Le dispositif 22 est du type permettant l'augmentation du pH de la solution dans laquelle il est disposé selon les réactions (3) décrites précédemment, des enzymes uréase étant disposées dans les fibres 26.

On dispose en outre, dans l'enveloppe 71, le composé Z, tel que précédemment décrit, susceptible de se solubiliser en milieu acide. Le seuil de coupure des fibres 26 permet d'empêcher le passage des substances A et Z, et des enzymes uréase. Le seuil de coupure de la membrane 54 permet d'empêcher le passage des enzymes uréase mais permet le passage de la substance A. En outre, le seuil de coupure des fibres 26' permet d'empêcher le passage des substances A et Z, et des enzymes glucose oxydase. Le seuil de coupure de la membrane 54' permet d'empêcher le passage des enzymes glucose oxydase mais permet le passage de la substance A. Le seuil de coupure de l'enveloppe 71 permet d'empêcher le passage de la substance Z. Le seuil de coupure de la membrane 76 permet d'empêcher le passage de la substance A.

En fonctionnement normal, le moteur 75 est placé dans une solution contenant du glucose et de l'urée. Le seuil de coupure des fibres 26' est suffisamment élevé pour permettre le passage du glucose et le seuil de coupure des fibres 26 est suffisamment élevé pour permettre le passage de l'urée. En outre, le seuil de coupure de l'enveloppe 71 est suffisamment élevé pour permettre le passage du solvant, du glucose et de l'urée. Les concentrations des substances A et Z sont initialement choisies de sorte que les pressions osmotiques dans la chambre de dilatation 60 et dans l'enveloppe 71 s'équilibrent, le piston 58 restant fixe.

Un cycle de fonctionnement du moteur 75 va maintenant être décrit.

La figure 5A représente le moteur 75 au début d'un cycle. Le piston 58 est en position de repos, le volume de la chambre de dilatation 60 étant minimal. La vanne d'évacuation 73 est fermée, la vanne 69' est fermée et la vanne 69 est ouverte. Dès que le moteur 75 est placé dans la solution contenant de l'urée, l'urée traverse l'enveloppe 71 et se répand jusque dans les fibres 26. La dégradation de l'urée conduit à l'obtention d'ions OH- qui se répandent dans l'enveloppe 71. L'augmentation du pH dans l'enveloppe 71 favorise la précipitation de la substance Z ce qui tend à faire baisser la pression osmotique dans l'enveloppe 71. La variation de pression osmotique entraîne un flux de liquide de l'intérieur de l'enveloppe 71 vers la chambre de dilation 60 par l'intermédiaire des fibres 26, déplaçant ainsi le piston 58. Le déplacement du piston 58 tend le ressort 72, permettant d'emmagasiner de l'énergie mécanique.

Sur la figure 5B, la chambre de dilatation 60 est représentée en expansion maximale. La vanne 69 est alors fermée et la vanne 69' est ouverte. En outre, la vanne d'évacuation 73 est ouverte. La pression à l'intérieur de la chambre de dilatation 60 s'égalise avec la pression du milieu ambiant. Le ressort 72 ramène le piston 58 en position de repos en évacuant, par la vanne d'évacuation 73, le solvant de la chambre de dilatation 60 dans le milieu ambiant. L'énergie mécanique emmagasinée dans le ressort 72 est ainsi récupérée. Par ailleurs, la vanne 69' étant ouverte, du glucose pénètre dans les fibres 26'. La dégradation du glucose conduit à l'obtention d'ions H⁺ qui se répandent dans l'enveloppe 71. La baisse du pH dans l'enveloppe 71 favorise la solubilisation de la substance Z, ce qui permet de retrouver les concentrations de la substance Z du début du cycle. La vanne 73 est finalement fermée, achevant ainsi le cycle moteur.

Dans le présent exemple de réalisation, la chambre de dilatation 60 est définie par un corps cylindrique 56 dans lequel coulisse un piston 58. En fonction de l'utilisation souhaitée du moteur 75 selon l'invention, la chambre de dilatation 60 peut être réalisée de façon différente.

Les figures 6A et 6B représentent un moteur osmotique 75' mettant en oeuvre une variante de structure de la chambre de dilatation 60 du moteur 75 de l'exemple de réalisation précédent. Selon cette variante, la chambre de dilatation 60 correspond à l'espace défini entre une enveloppe intérieure 77 et une enveloppe extérieure 78 à la manière d'une chambre à air. L'enveloppe intérieure 77 est déformable et extensible et entoure un corps déformable 79. L'enveloppe extérieure 78 est souple et inextensible. Elle se referme sur l'enveloppe intérieure 77 et est reliée au corps cylindrique 56. A titre d'exemple, dans une application médicale du moteur osmotique 75' selon l'invention, le corps déformable 79 peut être un organe creux du corps humain, tel que l'estomac, l'urètre, l'anus ou le coeur et les enveloppes 77, 78 peuvent définir des chambres de dilatation 60 en forme de boudins entourant l'organe creux. Le moteur osmotique joue alors le rôle d'une source motrice d'un sphincter artificiel (lorsque l'organe creux est l'urètre ou l'anus) ou d'un anneau gastrique commandable (lorsque l'organe creux est l'estomac).

Un cycle du moteur 75' selon la présente variante va maintenant être décrit.

La figure 6A représente le moteur 75' en début de cycle. Le volume de la chambre de dilatation 60 est minimal, le corps déformable 79 étant en dilation maximale, ce qui peut correspondre à un urètre ou à un canal anal ou à un estomac "ouverts" au maximum ou à un coeur en diastole. La vanne d'évacuation 73 est fermée, la vanne 69' est fermée et la vanne 69 est ouverte. Les enzymes uréase favorisent la dégradation de l'urée et l'augmentation du pH dans l'enveloppe 71, ce qui favorise la précipitation de la substance Z. Ceci entraîne, par osmose l'introduction de solvant dans la chambre de dilatation 60. L'enveloppe intérieure 77 se déforme et comprime le corps déformable 79.

Sur la figure 6B, le corps déformable 79 est comprimé au maximum, ce qui peut correspondre à un urètre "fermé", à un canal anal "fermé", à un estomac "ouvert" au minimum ou à un coeur en systole. La vanne 69' est alors ouverte et la vanne 69 est fermée. A l'ouverture de la vanne d'évacuation 73, le solvant évacue la chambre de dilatation 60 permettant la dilation du corps déformable 79, ce qui achève le cycle.

Selon une autre variante de l'invention, la chambre de dilatation est constituée d'une enveloppe supplémentaire élastique enfermant des fibres qui sont disposées, par exemple, en spirale et qui sont reliées à une extrémité au corps cylindrique 56. Lorsque du solvant pénètre dans les fibres, celles-ci ont tendance à se redresser et à déformer l'enveloppe élastique. A l'ouverture de la vanne 73, la pression à l'intérieur des fibres diminue et l'enveloppe supplémentaire tend à reprendre sa forme initiale.

Selon une autre variante de l'invention, le dispositif de variation de pH peut être relié à la chambre déformable par un conduit souple. Ceci permet de disposer de façon avantageuse le dispositif de variation de pH dans un milieu ambiant propice pour l'apport en solvant dans lequel sont dissoutes les substances utilisées (glucose ou urée par exemple) par les enzymes contenues dans le dispositif de variation de pH, et de placer la chambre de dilatation à un endroit où l'on souhaite disposer de l'énergie mécanique. Dans le cas d'une application médicale, on pourra disposer le dispositif de variation de pH dans un tissu graisseux, ou sur le réseau vasculaire. Dans ce dernier cas, les fibres peuvent être agencées pour former un tube creux, laissant en son centre un espace cylindrique permettant la circulation d'un fluide tel que le sang. Les bagues de jonction peuvent être de forme torique et placées contre la paroi d'un vaisseau sanguin. L'une des bagues de jonction toriques communique avec la chambre de dilatation par le conduit souple qui perfore le vaisseau sanguin.

Les figures 7A et 7B représentent une variante du dispositif 10 de variation de pH selon le premier exemple de réalisation de l'invention. Selon une telle variante, on prévoit dans l'enceinte 12 un piston mobile 81 adapté à coulisser dans l'enceinte 12. Le piston mobile 81 est entraîné par un moteur (M) 82, par exemple le moteur osmotique 70 décrit en relation aux figures 4A à 4D. La référence 84 désigne un élément de liaison entre le piston mobile 81 et le moteur 82, par exemple un moyen de rappel. Le piston mobile 81 et l'enceinte 12 définissent une chambre de volume variable 86. Une conduite d'évacuation 88 est reliée à la vanne 16.

La réaction privilégiée dans la chambre 86 est la réaction d'augmentation du pH par dégradation de l'urée (réactions (3)). Des enzymes uréase sont alors disposées dans la chambre 86. Les membranes 18, 20 ont un seuil de coupure permettant de retenir les enzymes uréase dans la chambre 86. Le dispositif 80 est adapté à une application selon laquelle, parallèlement à l'augmentation du pH, on souhaite éliminer de l'urée en excès dans la solution dans laquelle le dispositif 80 est placé.

Un cycle de fonctionnement du dispositif 80 selon l'invention va maintenant être décrit.

La figure 7A représente le dispositif 80 au début d'un cycle. Le volume de la chambre 86 est maximum, la vanne 14 est ouverte et la vanne 16 est fermée. La solution contenue dans la chambre 86 correspond sensiblement à la solution dans laquelle le dispositif 80 est disposé. La vanne 14 est alors fermée. La réaction de dégradation de l'urée se produit dans la chambre 86 entraînant une augmentation du pH de la solution contenue dans la chambre 86. Le moteur 82 est alors actionné pour déplacer le piston 81 entraînant une diminution du volume de la chambre 86. La vanne 16 est ouverte lors du déplacement du piston 81 entraînant l'expulsion de la solution contenue dans la chambre 86 par l'intermédiaire de la conduite 88.

La figure 7B représente le dispositif 80 à la fin de la phase ascendante du piston 81. La chambre 86 a alors un volume minimum. La vanne 16 est alors fermée. Le moteur 82 est alors actionné pour déplacer le piston 81 entraînant une augmentation du volume de la chambre 86. La vanne 14 est ouverte lors du déplacement du piston 81 entraînant la succion dans la chambre 86 de la solution dans laquelle est placé le dispositif 80. Le cycle s'achève lorsque la chambre 86 à un volume maximum.

Un tel dispositif 80 peut être implanté dans le corps humain pour l'élimination d'un excès d'urée. Les ions ammonium obtenus lors de la dégradation de l'urée sont alors susceptibles de réagir avec des éléments tels que le magnésium, le calcium et le potassium présents dans une solution biologique pour former des composés solides. Il peut alors ne pas être souhaitable de rejeter de tels composés solides hors du dispositif 80 dans la solution biologique. Pour ce faire, la conduite 88 est reliée à une région susceptible de recevoir les déchets issus de la dégradation de l'urée, par exemple au niveau du colon.

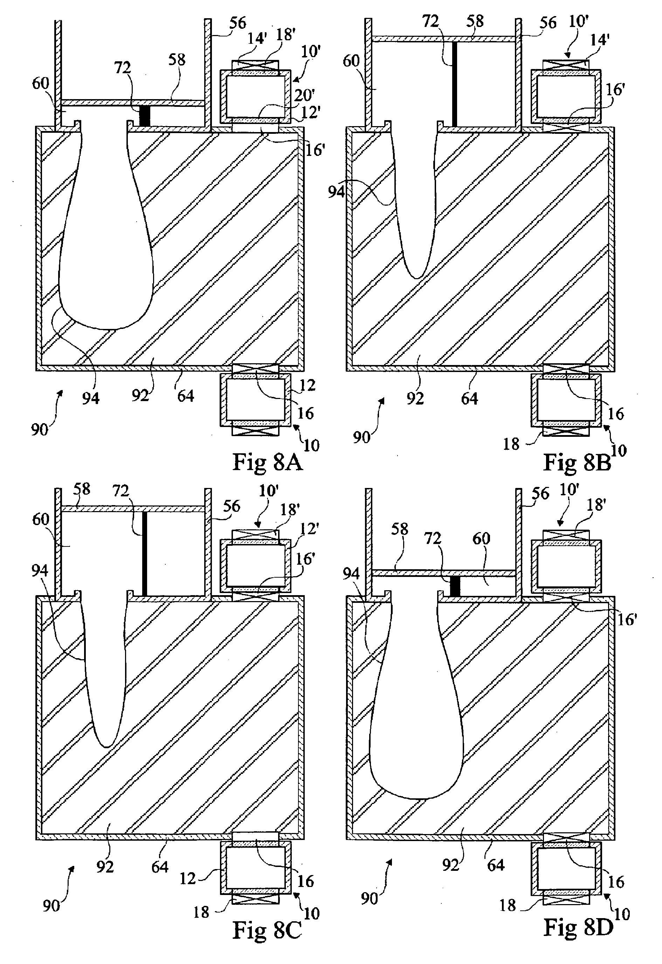

Les figures 8A à 8D représentent un troisième exemple de réalisation d'un moteur osmotique 90 selon l'invention. Le moteur osmotique 90 inclut certains composants du premier exemple de moteur osmotique 70 représenté aux figures 4A à 4D et les références correspondantes sont conservées.

Par rapport au moteur osmotique 70, l'enveloppe 52 et la membrane 54 ne sont pas présentes. Le boîtier 64 contient un gel polymère 92 capable de changer de volume en fonction du pH. Il s'agit par exemple de gel polymère réversible en fonction du pH tel que décrit dans les travaux de Y. Osada (Polymer Gels and Networks, Yoshihito Osada and Alexi R. Khokhlov, New York, Marcel Dekker Inc., 2001, 400 p.) qui augmente de volume lorsque le pH augmente. Une enveloppe étanche 94 est disposée dans le boîtier 92 et communique avec la chambre de dilatation 60. L'enveloppe 94 et la chambre de dilatation 60 sont remplies d'un solvant, par exemple une solution aqueuse.

Un cycle de fonctionnement du moteur 90 va maintenant être décrit.

La figure 8A représente le moteur 90 au début d'un cycle. Le gel 92 occupe un volume minimum. L'enveloppe 94 a donc un volume maximum et la chambre 60 a un volume minimum. Parallèlement au cycle d'ouvertures des vannes 16 et 16' qui va maintenant être décrit, les dispositifs 10, 10' sont commandés pour que les enceintes 12, 12' contiennent des solutions ayant le pH désiré.

La vanne 16 est fermée et la vanne 16' est ouverte. Les ions OH- se répandent dans le boîtier 64 entraînant une augmentation du pH. Le gel 92 augmente alors de volume et comprime l'enveloppe 94. Lorsque le pH dans l'enveloppe 52 a augmenté jusqu'à la valeur souhaitée, la vanne 16' est fermée. La diminution du volume de l'enveloppe 94 entraîne un transfert de solvant de l'enveloppe 94 vers la chambre de dilatation 60, entraînant le déplacement du piston 58. Le piston 58 est en phase ascendante.

La figure 8B représente le moteur 90 à la fin de la phase ascendante du piston 58. La chambre 60 a alors un volume maximum.

En figure 8C, on a ouvert la vanne 16 de façon à mettre en communication le contenu du boîtier 64 avec le contenu de l'enceinte 12. Des ions H⁺ se répandent dans le boîtier 64 entraînant une diminution du pH dans le boîtier 64. Lorsque le pH est suffisamment acide, la vanne 16 est fermée. La diminution du volume du gel 92 est alors favorisée, entraînant l'augmentation du volume de l'enveloppe 94. Un nouveau transfert de solvant se produit, du solvant passant de la chambre de dilatation 60 vers l'enveloppe 94. L'action du ressort 72 favorise l'évacuation du solvant de la chambre de dilatation 60. Toutefois, le ressort 72 pourrait ne pas être présent, le piston 58 se déplaçant seulement par succion. Le piston 58 est dit en phase descendante.

La figure 8D représente le moteur 90 à la fin de la phase descendante du piston 58, ce qui clôt le cycle.

L'utilisation du gel 92 dont le volume varie en fonction du pH peut être mise en oeuvre pour la réalisation d'un actionneur à coup unique. A titre d'exemple, par rapport au moteur 90, seul le dispositif 10' est présent. Le fonctionnement de l'actionneur est alors identique à ce qui a été décrit précédemment pour le moteur 90 en relation aux figures 8A et 8B. Selon un autre exemple, par rapport au moteur 90, seul le dispositif 10 est présent. Le fonctionnement de l'actionneur est alors identique à ce qui a été décrit précédemment pour le moteur 90 en relation aux figures 8C et 8D.

Selon une variante des actionneurs et moteurs osmotiques 70, 90 décrits précédemment, les membranes 20, 20' et 54 peuvent être remplacées par un faisceau de fibres ou un système analogue s'il est souhaitable d'augmenter la surface d'échange entre le contenu de l'enceinte 12, 12' et le contenu du boîtier 64 ou entre le contenu de l'enveloppe 52 et le contenu de la chambre de dilatation 60.

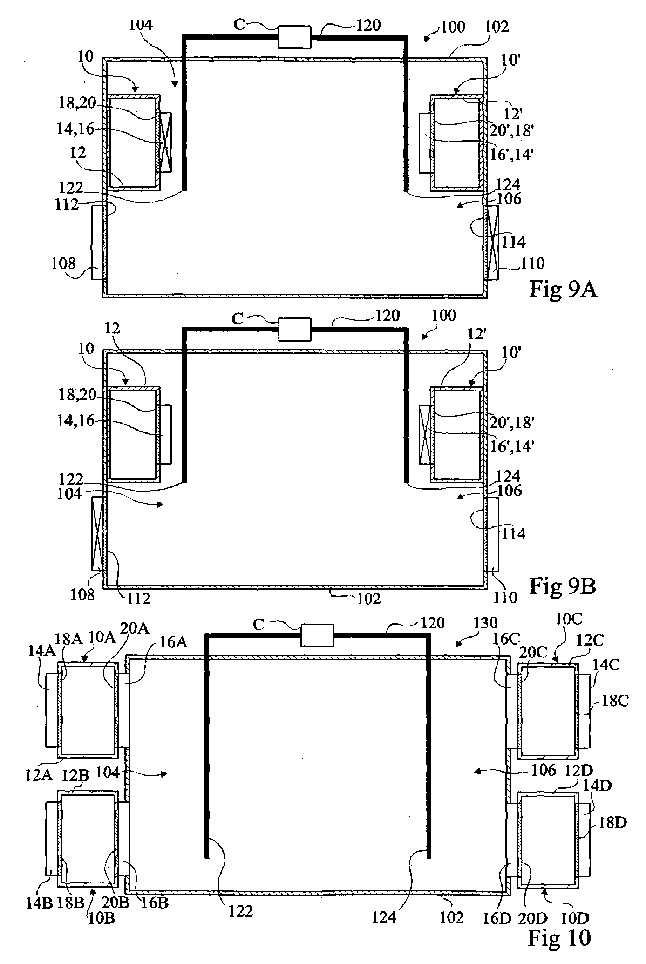

Les figures 9A et 9B représente un premier exemple de réalisation d'une pile 100 selon l'invention mettant en oeuvre le premier exemple de dispositif de variation de pH. La pile 100 est destinée à fonctionner dans une solution biologique. Elle comprend une enceinte étanche 102 comprenant deux régions 104, 106 qui communiquent entre elles. Une vanne 108, lorsqu'elle est ouverte, met en communication le contenu de l'enceinte 102, du côté de la région 104, avec la solution extérieure. Une vanne 110, lorsqu'elle est ouverte, met en communication le contenu de l'enceinte 102, du côté de la région 106, avec la solution extérieure. La pile 100 comprend une membrane 112 disposée entre la vanne 108 et l'enceinte 102 et une membrane 114 disposée entre la vanne 110 et l'enceinte 102.

La pile 100 comprend un premier dispositif de variation de pH 10, disposé au niveau de la région 104, pour lequel, par rapport à ce qui est représenté en figure 1, les vannes 14 et 16 sont confondues et les membranes 18, 20 sont confondues. En outre, la pile 100 comprend un second dispositif de variation de pH, disposé au niveau de la région 106. Dans la suite de la description, on utilise des références primes pour désigner les éléments du second dispositif de variation du pH de façon à les distinguer des éléments du premier dispositif de variation de pH 10. Les dispositifs 10 et 10' sont du type permettant la diminution du pH de la solution qu'il contient. A titre d'exemple, les dispositifs 10, 10' permettent une acidification de la solution contenue dans l'enceinte 12, 12' associée selon les réactions (1) précédemment décrites.

Un élément conducteur 120 relie la région 104 de l'enceinte 102 et la région 106 de l'enceinte 102 par l'intermédiaire d'une charge C destinée à être alimentée par la pile 100. L'élément conducteur 120 se prolonge par une électrode 122 qui peut par exemple être en hydroxyde ferrique Fe(OH)₃ dans la région 104 de l'enceinte 102 et par une électrode 124 en hydroxyde ferrique dans la région 106 de l'enceinte 102.

La pile 100 est placée dans une solution contenant du D-glucose, par exemple une solution biologique, et dont le pH est sensiblement neutre ou éventuellement légèrement acide ou basique. Les membranes 112, 114 sont adaptées à laisser passer le D-glucose, le gluconate. Toutefois, les membranes 112, 114 ne laissent pas passer les ions positifs. Pour ce faire, les membranes 112, 114 peuvent être chargées positivement. Un exemple de membranes chargées positivement, ce qui peut être obtenu par des techniques telles que celles utilisées par la société ASTOM. Les membranes 112, 114 laissent donc passer les ions négatifs de petites dimensions, tels que les ions hydroxyles OH⁻ et les ions bicarbonate HCO₃⁻.

Selon une variante, les membranes 20, 20', 112 et 114 peuvent être remplacées par un faisceau de fibres ou un système analogue s'il est souhaitable d'augmenter la surface d'échange entre le contenu de l'enceinte 12, 12' et le contenu du boîtier 102 ou entre le contenu du boîtier 102 et le milieu extérieur. En outre, plusieurs vannes (et les membranes associées) commandées de la même façon que la vanne 108 peuvent être réparties sur le boîtier 102 au niveau de la région 104. De façon analogue, plusieurs vannes (et les membranes associées) commandées de la même façon que la vanne 110 peuvent être réparties sur le boîtier 102 au niveau de la région 106. En outre, plusieurs vannes (et les membranes associées) commandées de la même façon que la vanne 14 peuvent être réparties sur l'enceinte 12 du dispositif 10. De façon analogue, plusieurs vannes (et les membranes associées) commandées de la même façon que la vanne 14' peuvent être réparties sur l'enceinte 12' du dispositif 10'.

La figure 9A représente la pile 100 dans une première phase de fonctionnement. La vanne 108 est ouverte et la vanne 110 est fermée. En outre, la vanne 14 est fermée et la vanne 14' est ouverte. Du D-glucose pénètre donc dans l'enceinte 12' du dispositif 10'. Les réactions (1) décrites précédemment, qui se déroulent dans l'enceinte 12', entraînent la libération d'ions H⁺ qui se répandent à l'extérieur du dispositif 10', dans la région 106 de l'enceinte 102, et en particulier au niveau de l'électrode 124. Le fonctionnement de la pile est basé sur des réactions d'oxydoréduction qui sont plus ou moins favorisées en fonction du pH. Plus précisément, dans la première phase de fonctionnement, l'électrode 124 joue le rôle de cathode. La présence d'ions H⁺ au niveau de la cathode 124 favorise la réaction de réduction suivante :

Fe(OH}₃ + 3H⁺ + e⁻ →Fe²⁺ + 3H₂O (4)

La vanne 14 étant fermée, le dispositif 10 ne libère pas d'ions H⁺ dans la région 104 de l'enceinte 102. En outre, la vanne 110 étant fermée et la vanne 108 étant ouverte, on favorise l'établissement d'un gradient de pH entre la région 104 et la région 106 de l'enceinte 102, le pH étant plus élevé dans la région 104 que dans la région 106. En effet, les ions H⁺ qui se propagent jusqu'à la région 104 tendent à réagir par exemple avec des ions hydroxyle OH- présents dans la solution biologique et traversant la membrane 112 pour former de l'eau ou avec des ions bicarbonate HCO₃⁻ pour former de l'eau et du dioxyde de carbone.

Les ions Fe²⁺, qui se forment à la cathode 124, sont piégés dans l'enceinte 102 et migrent jusqu'à l'électrode 122 qui joue le rôle d'anode. La communication avec l'extérieur par la vanne 108 permet au pH de rester proche de la neutralité, ce qui favorise, à l'anode 122, la réaction d'oxydation suivante :

Fe²⁺ +3H₂O→Fe(OH)₃ +3H⁺ +e⁻ (5)

On observe donc un transfert d'électrons de l'anode 122 vers la cathode 124 à travers l'élément conducteur 120 et donc à travers la charge C à alimenter.

La réaction (4) entraîne donc la consommation de l'hydroxyde ferrique Fe(OH)₃ de la cathode 124. Pour permettre un fonctionnement à long terme de la pile 100, il est avantageux de prévoir une inversion régulière de la polarité de la pile 100.

La figure 9B représente la pile 100 dans une seconde phase de fonctionnement. La vanne 108 est fermée et la vanne 110 est ouverte. En outre, la vanne 14 est ouverte et la vanne 14' est fermée. Les réactions (1) décrites précédemment qui se déroulent dans l'enceinte 12 entraînent la libération d'ions H⁺ qui se répandent à l'extérieur du dispositif 10, dans la région 104 de l'enceinte 102, et en particulier au niveau de l'électrode 122 qui joue alors le rôle de cathode. La présence d'ions H⁺ au niveau de la cathode 122 favorise la réaction de réduction (4) décrite précédemment.

La vanne 14' étant fermée, le dispositif 10' ne libère pas d'ions H⁺ dans la région 106 de l'enceinte 102. En outre, la vanne 108 étant fermée et la vanne 110 étant ouverte, on favorise l'établissement d'un gradient de pH entre la région 106 et la région 104 de l'enceinte 102, le pH étant plus élevé dans la région 106 que dans la région 104. En effet, les ions H⁺ qui se propagent jusqu'à la région 106 tendent à réagir par exemple avec des ions hydroxyle OH- présents dans la solution biologique et traversant la membrane 114 pour former de l'eau ou avec des ions bicarbonate HCO₃⁻ pour former de l'eau et du dioxyde de carbone.

Les ions Fe²⁺, qui se forment à la cathode 122, sont piégés dans l'enceinte 102 et migrent jusqu'à l'électrode 124 qui joue alors le rôle d'anode. On favorise donc, à l'anode 124, la réaction d'oxydation (5) décrite précédemment.

On observe donc un transfert d'électrons de l'anode 124 vers la cathode 122 à travers l'élément conducteur 120 et donc à travers la charge C à alimenter.

Les phases de fonctionnement décrites précédemment en relation avec les figures 9A et 9B sont alternées pour permettre une reconstitution du stock de Fe(OH)₃ au niveau des électrodes 122, 124.

La figure 10 représente un second exemple de réalisation d'une pile 130 selon l'invention mettant en oeuvre le premier exemple de dispositif de variation de pH. La pile 130 peut fonctionner sans être immergée dans une solution biologique. Par rapport à la pile 100, la pile 130 comprend quatre dispositifs de variation de pH tels que décrits précédemment en relation avec la figure 1. Dans la suite de la description, on ajoute les suffixes "A", "B", "C" et "D" pour désigner les éléments associés respectivement aux dispositifs de variation de pH 10A, 10B, 10C et 10D. Les vannes 16A et 16B des dispositifs 10A, 10B débouchent au niveau de la région 104 de l'enceinte 102 et les vannes 16C et 16D des dispositifs 10C, 10D débouchent au niveau de la région 106 de l'enceinte 102.

Les dispositifs 10A et 10C sont du type permettant la diminution du pH de la solution qu'ils contiennent. A titre d'exemple, les dispositifs 10A et 10C permettent une acidification de la solution contenue dans l'enceinte 12A, 12C associée selon les réactions (1) décrites précédemment. Les dispositifs 10B et 10D sont du type permettant l'augmentation du pH de la solution qu'ils contiennent. A titre d'exemple, les dispositifs 10B, 10D permettent une alcalinisation de la solution contenue dans l'enceinte 12B, 12D associée selon les réactions (3) décrites précédemment.

Un exemple de fonctionnement de la pile 130 est le suivant. Parallèlement au cycle d'ouvertures des vannes 16A à 16D qui va maintenant être décrit, les dispositifs 10A à 10D sont commandés pour que les enceintes 12A à 12D associées contiennent des solutions ayant le pH désiré. Pendant une première phase de fonctionnement, la vanne 16A est ouverte, la vanne 16B est fermée, la vanne 16C est fermée et la vanne 16D est ouverte. Des ions H⁺ sont alors libérés par le dispositif 10A entraînant une diminution du pH au niveau de l'électrode 122 et des ions OH- sont libérés par le dispositif 10D entraînant une augmentation du pH au niveau de l'électrode 124. Un gradient de pH est alors obtenu entre les régions 104 et 106 de l'enceinte 102. La présence d'ions H⁺ au niveau de l'électrode 122 favorise la réaction de réduction (4) décrite précédemment et la présence d'ions OH- au niveau de l'électrode 124 favorise la réaction d'oxydation (5) décrite précédemment. On observe donc un transfert d'électrons de l'anode 124 vers la cathode 122 à travers l'élément conducteur 120 et donc à travers la charge C à alimenter. Pendant une seconde phase de fonctionnement, la vanne 16A est fermée, la vanne 16B est ouverte, la vanne 16C est ouverte et la vanne 16D est fermée. Des ions H⁺ sont alors libérés par le dispositif 10C entraînant une diminution du pH au niveau de l'extrémité 124 et des ions OH- sont libérés par le dispositif 10B entraînant une augmentation du pH au niveau de l'électrode 122. La présence d'ions H⁺ au niveau de l'électrode 124 favorise la réaction de réduction (4) décrite précédemment et la présence d'ions OH⁻ au niveau de l'électrode 122 favorise la réaction d'oxydation (5) décrite précédemment. On observe donc un transfert d'électrons de l'anode 122 vers la cathode 124 à travers l'élément conducteur 120 et donc à travers la charge C à alimenter.

Dans les exemples de piles décrits précédemment, il est possible d'utiliser, au lieu d'électrodes en hydroxyde ferrique, plusieurs sortes d'électrodes à condition que leur potentiel dépende du pH. Le potentiel d'électrode étant imposé par l'échange électronique entre les deux formes d'un couple oxydant-réducteur, il est possible d'envisager différentes constitutions pour les électrodes : les deux formes peuvent être solubles, une forme peut être soluble, l'autre insoluble, et, enfin, les deux formes peuvent être insolubles. Dans ce dernier cas, il peut être avantageux d'utiliser des électrodes constituées d'un polymère électro-actif obtenu par exemple à partir de condensation de phénols ou de thiols. Un exemple de polymère ayant une capacité redox est décrit dans le document Ion Exchange, Friedrich Helfferich, 1962 McGraw-Hill Book Company, Chapitre 12 : Electron Exchangers and Redox Ion Exchangers, pages 551 à 568.

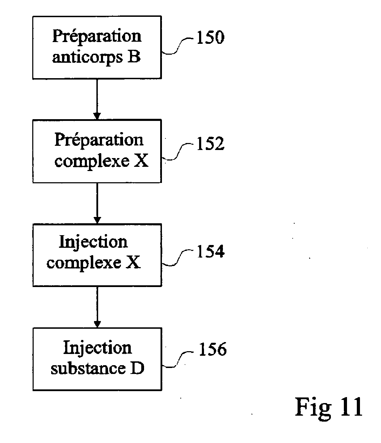

La figure 11 illustre les étapes d'un exemple de procédé de thérapie enzymatique ciblée. Un exemple de procédé de thérapie enzymatique ciblée est décrit dans le document "Lysosomal Enzyme Delivery by ICAM-1-Targeted Nanocarriers Bypassing Glycosylation- and Clathrin-Dependent Endocytosis" aux noms de Sylvia Muro, Edward H. Schuchman and Vladimir R. Muzykantov (Molecular Therapy vol.13, n°1, janvier 2006, pages 135 à 140).

A l'étape 150, on détermine un anticorps B monoclonal reconnaissant un antigène A de la cellule cible. A titre d'exemple, l'antigène A correspond à l'antigène carcino-embryonnaire ou antigène ACE (correspondant à l'anglais carcinoembryonic antigen ou CEA), l'antigène prostatique spécifique (correspondant à l'anglais Prostate Specific Antigen ou PSA), ou l'antigène de membrane spécifique de la prostate (correspondant à l'anglais Prostate Specific Membrane Antigen ou PSMA). Un anticorps B adapté à l'antigène PSA est, par exemple, l'anticorps commercialisé par la société Cytrogen Corporation sous la désignation commerciale Prostascint (Capromab Pendetide).

A l'étape 152, on forme un complexe X combinant l'anticorps monoclonal B et une enzyme G. L'enzyme G est adaptée à favoriser une réaction mettant en oeuvre une substance D. Le complexe X est tel que l'affinité de l'anticorps B avec l'antigène A reste forte et que l'activité de l'enzyme G sur la substance D reste également forte. La substance D est, par exemple, le L-glucose et l'enzyme G correspond à une enzyme L-glucose oxydase, par exemple la D-threo-aldose 1-dehydrogenase. Une telle enzyme présente l'avantage d'être inactive sur le D-glucose. Selon un autre exemple, la substance D est le mannitol (C₆H₈(OH)₆) et l'enzyme G correspond à une enzyme mannitol oxydase.

A l'étape 154, on injecte le complexe X dans le corps du patient. L'injection du complexe X est, par exemple, réalisée par voie intraveineuse. Le complexe X tend alors à se fixer sur l'antigène A par l'intermédiaire de l'anticorps B. On attend alors que le complexe X circulant dans le corps du patient soit éliminé.

A l'étape 156, on injecte la substance D dans le corps du patient. L'injection de la substance D est, par exemple, réalisée par voie intraveineuse. Dans le cas où l'enzyme G est une L-glucose oxydase, on injecte du L-glucose dans le corps du patient. Le L-glucose diffuse alors librement dans l'organise. Le L-glucose étant inoffensif pour le corps humain, la concentration de L-glucose peut être élevée (par exemple de l'ordre de la concentration en glucose du sang, soit plusieurs milli-moles par litre). Dans le cas où l'enzyme G est une mannitol oxydase, on injecte du mannitol dans le corps du patient, par exemple le stéréoisomère D du mannitol.

Dans le cas d'injection de L-glucose, les cellules humaines n'étant pas adaptées à métaboliser le L-glucose, seules les enzymes G des complexes X vont favoriser les réactions (2) décrites précédemment, entraînant, à partir du L-glucose, la production d'ions H⁺ et, par conséquent, une diminution locale du pH. Cette diminution locale du pH peut se traduire par un effet thérapeutique direct entraînant la mort de la cellule cible ou peut entraîner une réaction inflammatoire entraînant la mort de la cellule cible.

Dans le cas d'injection de mannitol, les enzymes G favorisent la réaction dans laquelle le mannitol réagit avec du dioxygène pour fournir du mannose (C₆H₇O(OH)₅) et du peroxyde d'hydrogène, ou eau oxygénée (H₂O₂), selon la réaction suivante :

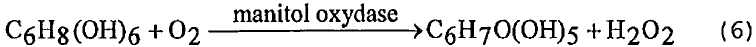

La production locale d'eau oxygénée peut se traduire par un effet thérapeutique direct entraînant la mort de la cellule cible ou peut entraîner une réaction inflammatoire entraînant la mort de la cellule cible.

Un aspect de la présente invention prévoit donc un procédé de traitement comprenant les étapes consistant à former un complexe comprenant un anticorps d'un antigène d'une cellule cible et une enzyme adaptée à favoriser une réaction qui produit une seconde substance à partir d'une première substance, la seconde substance étant nocive pour la cellule cible ; introduire le complexe dans le corps du patient d'où il résulte que l'anticorps du complexe se fixe sur la cellule cible ; et introduire la première substance dans le corps du patient d'où il résulte que l'enzyme du complexe favorise la production de la seconde substance au niveau de la cellule cible.

Selon un mode de réalisation, la première substance est le L-glucose, l'enzyme est l'enzyme L-glucose oxydase et la seconde substance est l'ion H⁺.

Selon un mode de réalisation, la première substance est le mannitol, l'enzyme est l'enzyme mannitol oxydase et la seconde substance est l'eau oxygénée.

Selon un mode de réalisation, l'antigène est un antigène parmi le groupe comprenant l'antigène carcino-embryonnaire, l'antigène prostatique spécifique ou l'antigène de membrane spécifique de la prostate.

Bien entendu, la présente invention est susceptible de diverses variantes et modifications qui apparaîtront à l'homme de l'art. En particulier, la variante de réalisation de la chambre de dilatation du moteur osmotique décrit en relation aux figures 6A et 6B peut être appliquée au moteur osmotique décrit en relation aux figures 4A à 4D.

## Revendications

1. Pile (100 ; 130) comprenant :
une enceinte (102) contenant des première et seconde électrodes (122, 124), une réaction de réduction ou d'oxydation étant susceptible de se produire au niveau de la première et de la seconde électrode en fonction du pH ;
un dispositif (10, 10' ; 10A, 10B, 10C, 10D) de variation de pH d'un solvant dans lequel est dissoute une substance, l'enceinte (12 ; 24) étant en contact avec le solvant par l'intermédiaire d'une paroi d'enceinte (18 ; 26) perméable au solvant et à la substance, l'enceinte contenant des enzymes adaptées à transformer la substance pour fournir des ions hydrogène ou hydroxyle, la paroi d'enceinte étant non perméable aux enzymes, ce dispositif étant adapté à amener le pH au niveau de la première électrode dans une première plage de pH donnée favorisant l'une des réactions de réduction ou d'oxydation ; et
un moyen (108, 110 ; 10A, 10B, 10C, 10D) adapté à amener le pH au niveau de la seconde électrode dans une seconde plage de pH donnée différente de la première plage favorisant l'autre des réactions de réduction ou d'oxydation.

2. Pile selon la revendication 1, dans laquelle la pile est destinée à être disposée au contact d'un solvant, ledit moyen (108, 110) correspondant à une vanne susceptible de faire communiquer le contenu de l'enceinte (102) avec le solvant au niveau de la seconde électrode (122, 124).

3. Pile selon la revendication 2, dans laquelle le moyen (10A, 10B, 10C, 10D) est un dispositif supplémentaire de variation de pH.

## Claims

1. A cell (100; 130) comprising:
an enclosure (102) containing first and second electrodes (122, 124), a reduction or oxidation reaction being likely to occur at the level of the first and of the second electrode according to the pH;
a device (10, 10'; 10A, 10B, 10C, 10D) for varying the pH of a solvent in which a substance is dissolved, the enclosure (12; 24) being in contact with the solvent via an enclosure wall (18; 26) permeable to the solvent and to the substance, the enclosure containing enzymes capable of transforming the substance to release hydrogen or hydroxyl ions, the enclosure wall being non-permeable to enzymes, this device being capable of bringing the pH at the level of the first electrode within a given pH range promoting one of the reduction or oxidation reactions; and
means (108, 110; 10A, 10B, 10C, 10D) capable of bringing the pH at the level of the second electrode within a second given pH range different from the first range promoting the other one of the reduction or oxidation reactions.

2. The cell of claim 1, wherein the cell is intended to be arranged in contact with a solvent, said means (108, 110) corresponding to a valve likely to have the content of the enclosure (102) communicate with the solvent at the level of the second electrode (122, 124).

3. The cell of claim 2, wherein the means (10A, 10B, 10C, 10D) are an additional device for varying the pH.

## Patentansprüche

1. Eine Zelle (100; 130), die Folgendes aufweist:
eine Umschließung (102), die erste und zweite Elektroden (122, 124) enthält, wobei eine Reduktions- oder Oxidations-Reaktion mit Wahrscheinlichkeit auf dem Niveau der ersten und der zweiten Elektrode entsprechend dem pH-Wert auftritt;
eine Vorrichtung (10, 10'; 10A, 10B, 10C, 10D) zur Veränderung des pH-Werts eines Lösungsmittels in dem eine Substanz aufgelöst ist, wobei die Umschließung (12; 24) in Kontakt mit dem Lösungsmittel steht, und zwar über eine Umschließungswand (18; 26) permeabel für das Lösungsmittel und die Substanz, wobei die Umschließung Enzyme enthält, die in der Lage sind, die Substanz zu transformieren um Wasserstoff- oder Hydroxyl-lonen freizusetzen, wobei die Umschließungswand für Enzyme nicht permeabel ist und wobei diese Vorrichtung in der Lage ist, den pH-Wert auf ein Niveau der ersten Elektrode zu bringen, und zwar innerhalb eines gegebenen pH-Bereichs, was eine der Reduktions- oder Oxidations-Reaktionen fördert; und
Mittel (108; 110; 10A, 10B, 10C, 10D), die in der Lage sind, den pH-Wert auf ein Niveau der zweiten Elektrode zu bringen, und zwar innerhalb eines zweiten gegebenen pH-Bereichs der unterschiedlich von dem ersten Bereich ist, und zwar zur Förderung der anderen Reaktion der Reduktions- oder Oxidations-Reaktionen.

2. Die Zelle nach Anspruch 1, wobei die Zelle vorgesehen ist zur Anordnung in Kontakt mit einem Lösungsmittel, wobei die Mittel (108, 110) einem Ventil entsprechen, das mit Wahrscheinlichkeit den Inhalt der Umschlie-βung (102) mit dem Lösungsmittel verbindet auf dem Niveau der zweiten Elektrode (122, 124).

3. Die Zelle nach Anspruch 2, wobei die Mittel (10A, 10B, 10C, 10D) eine zusätzliche Vorrichtung zur Veränderung des pH-Werts sind.
